# EUROPEAN PATENT SPECIFICATION

(11) **EP 1 551 344 B1**
(45) Date of publication and mention of the grant of the patent: **07.01.2009**
(21) Application number: 03755512.5
(22) Date of filing: 28.05.2003
(51) Int. Cl.: A61F 13/15

(54) **ABSORBENT ARTICLE WITH MULTIPLE CORE**
SAUGFÄHIGER ARTIKEL MIT MEHRFACHKERN
ARTICLE ABSORBANT A AME MULTIPLE

(30) Priority: 28.05.2002 US 156385
(43) Date of publication of application: 13.07.2005
(73) Proprietor: Tyco Healthcare Retail Services AG, 8201 Schaffhausen (CH)
(72) Inventor: GLAUG, Frank, S., Chester Springs, PA 19425 (US); COLE, Robert, Theodore, Jackson, NJ 08527 (US); SERAFINO, Jean, Clifton Heights, PA 19018 (US)
(74) Representative: Frohwitter, Bernhard
(86) International application number: PCT/US2003/016678
(87) International publication number: WO 2003/099182

(56) References cited:
- EP-A- 0 998 893
- WO-A-98/43684
- US-A- 2 331 271
- US-A- 5 484 430
- US-A- 5 792 130

## Description

### BACKGROUND

### 1. Technical Field

The present disclosure generally relates to disposable absorbent articles, and more particularly, to disposable absorbent articles having multiple absorbent cores in fluid communication.

### 2. Background of the Related Art

Absorbent articles such as, for example, disposable diapers, adult incontinent pads, sanitary napkins, pantiliners, incontinent garments, etc. are generally worn, in cooperation with garments and disposed against a body surface, etc., by infants or adult incontinent individuals. The absorbent article is employed to collect, absorb, etc. body fluid discharge, such as, for example, blood, menses, urine, aqueous body fluids, mucus, cellular debris, etc. For example, the absorbent article may be disposed between the legs of an individual adjacent a crotch area. The absorbent article is positioned with a garment and drawn into engagement with a body surface of the crotch area to collect fluid discharge.

As is known, absorbent articles typically include a fluid permeable coverstock for engaging the body surface, a fluid impermeable back sheet and an absorbent core supported therebetween. The back sheet serves as a moisture barrier to prevent fluid leakage to the garment. The absorbent core usually includes a liquid retention material that faces the body surface. The absorbent core can include loosely formed cellulosic fibers, such as wood pulp, for acquiring and storing fluid discharge.

The absorbent core absorbs fluid discharge and with regard to adult absorbent articles, such as, incontinent pads, are made fairly thick to handle large quantities of fluid, such as urine. The absorbent cores, however, can be unseemly thick due to their size and bulk. One of the disadvantages of these absorbent articles is the thick, diaper-like appearance which may be embarrassing to an adult wearer.

More recently, to overcome bulkiness, other absorbent articles, particularly feminine pads, sanitary napkins, pantiliners, incontinent garments, etc., are manufactured as long, narrow and relatively flat. These absorbent articles are designed to be worn tightly against a contour of the body surface and held in place by an undergarment. Some of these designs, however, have a tendency to deform undesirably by, for example, bunching, roping, wrinkling, etc. as they conform to the contour of the body surface. Undesired deformity of the absorbent article may result in leakage of fluid discharge due to inefficiency in design.

Further, fluid discharge leakage typically results, not from over-saturation of the absorbent core, but from pooled fluid discharge run off. To wit, during a fluid discharge, such as a void, it is common for urine to deposit onto the coverstock to form a pool before it penetrates the absorbent core. If the absorbent article is undesirably deformed and pooling occurs, urine will not be absorbed because the core is bunched. Thus, run off occurs and premature leakage from the absorbent article results.

Attempts have been made to provide improved fitting absorbent articles. Some designs provide arcuate shaped pads incorporating elastic elements along longitudinal sides thereof. See, e. g., U. S. Patent Nos.: 4,701, 177 and 4,770, 657. Other designs attempt to prevent leakage by utilizing a pair of embossed rows along longitudinal edges of a top sheet. See, e. g., U. S. Patent No. 4,655, 759. Still further, other designs utilize an hour glass shape. See, e. g., U. S. Patent No. 5,032, 121. Designs of this type, however, do not adequately address product and absorbent core deformity to facilitate fluid management. These prior art designs may not adequately prevent undesirable deformity which can impede absorbency performance. Undesired product deformity may alter the desired shape of the absorbent article and cause premature leakage. WO 98/43684 describes an arrangement in which a sub-divided central absorbent core overlays a secondary storage region of cellulose material while EP 0 998 893 A2 describes an arrangement in which a central absorbent region is divided from end regions by a groove. Further arrangements of absorbent articles with grooves between absorbent sections are shown in JP 1-141707U and US 4,773,905.

It would therefore be desirable to overcome the disadvantages and drawbacks of the prior art by providing an absorbent article including multiple cores that are separate and in fluid communication. It is contemplated that the absorbent article would prevent undesired product deformity thereby improving absorbency performance. It is further contemplated that the absorbent article conforms to the contour of a body surface and allows for the quick dispersal of fluid discharge.

### SUMMARY

Accordingly, an absorbent article is disclosed which includes multiple cores that are separate and in fluid communication. The absorbent article is configured to prevent undesired product deformity for improved absorbency performance. The absorbent article can conform to the contour of a body surface and allows for the quick dispersal of fluid discharge through advantageous fluid management.

Objects and advantages of the present disclosure are set forth in part herein and in part will be obvious therefrom, or may be learned by practice of the present disclosure which is realized and attained by the instrumentalities and combinations pointed out in the appended claims for the devices and methods of the present disclosure consisting of its constituent parts, constructions, arrangements, combinations steps and improvements herein shown and described.

The absorbent article may include a plurality of compartments and core sections. The compartments and core sections may be evenly spaced and sandwiched between a top sheet and a back sheet. The core sections may include fluid retention materials, such as, for example, wood pulp fibers, super absorbent polymer particles, etc. It is contemplated that acquisition layers or elastics may be disposed along longitudinal sides of the absorbent article to facilitate formation of a reservoir with absorbent barriers so that large voids can be managed until the absorbent core can absorb excess fluid. The separate absorbent compartments and core sections cooperate with the elastics to provide spring-like hinges within the absorbent core to resist undesired deformity. This results in an absorbent article which folds in a desired configuration and provides comfort, improved absorbency protection and structural flexibility.

Advantageously, fluid permeable channels may be formed and separate the compartments and core sections to channel overflow fluid discharge from leaking from the absorbent article. The channels allow fluid to move throughout the absorbent material. This configuration advantageously improves fluid retention, results in less overflow and provides a more even distribution of fluids. The channels may also facilitate folding the absorbent article in a bucket-like configuration to create barriers between the compartments and core section.

In one particular embodiment, wich does not form part of the invention in accordance with the principles of the present disclosure, an absorbent article is provided which includes a fluid permeable top sheet and a first absorbent core that is separate and in fluid communication with a second absorbent core. The second absorbent core is separate and in fluid communication with a third absorbent core. The first, second and third absorbent cores are supported between the fluid permeable top sheet and a fluid impermeable back sheet. The second absorbent core defines at least two separate absorbent core sections in fluid communication. The separate absorbent core sections define a body facing surface that engages the fluid permeable top sheet. The absorbent cores may be separated by fluid flow channels. The absorbent core sections may also be separated by fluid flow channels.

In an embodiment, the absorbent article has a longitudinal length and includes a fluid permeable top sheet and a first absorbent core disposed adjacent a side of a central second absorbent core and pivotable, along the longitudinal length, relative to the second absorbent core. The first absorbent core is separate and in fluid communication with the second absorbent core. A third absorbent core is disposed adjacent to an opposing side of the second absorbent core and pivotable, along the longitudinal length, relative to the second absorbent core. The third absorbent core is separate and in fluid communication with the second absorbent core. The first, second and third absorbent cores are enclosed between the fluid permeable top sheet and a fluid impermeable back sheet. The second absorbent core has a mid-section and lateral sections disposed on opposing sides of the mid-section. The mid-section and lateral sections are separate, in fluid communication and define a body-facing surface that engages the fluid permeable top sheet. In another alternate embodiment, fluid flow channels are disposed between the absorbent cores and core sections for fluid management.

### BRIEF DESCRIPTION OF THE DRAWINGS

The objects and features of the present disclosure are set forth with particularity in the appended claims. The present disclosure, as to its organization and manner of operation, together with further objectives and advantages may be understood by reference to the following description, taken in connection with the accompanying drawings, in which:
FIG. 1 is a side perspective view of one particular embodiment of an absorbent article in accordance with the principals of the present disclosure;
FIG. 2 is a side view, in part perspective, of the absorbent article shown in FIG. 1;
FIG. 3 is a top view of the absorbent article shown in FIG. 1; and
FIG. 4 is a top view of an alternate embodiment of the absorbent article shown in FIG. 1.

### DETAILED DESCRIPTION OF EXEMPLARY EMBODIMENTS

The exemplary embodiments of the absorbent article and methods of use disclosed are discussed in terms of fluid absorbent articles, and more particularly, in terms of multiple absorbent core configurations that provide maximum protection and comfort to an individual wearing the absorbent article. It is contemplated that the absorbent article, in accordance with the principals of the present disclosure, prevents undesired product deformity, such as, for example, roping, bunching, breaks, wrinkling, etc. The presently disclosed article thereby avoids premature leakage, overflow, etc., of fluid discharge, such as, for example, blood, menses, urine, acqueous body fluids, mucus, cellular debris, etc. It is contemplated that the absorbent article may be employed with disposable diapers, adult incontinent pads, feminine pads, sanitary napkins, pantiliners, incontinent garments, etc. It is further contemplated that the present disclosure can also be used with bedding and furniture underpads, wound dressings, etc.

In the discussion that follows, the term "body facing surface" refers to a portion of a structure that is oriented towards a body surface, and the "garment facing surface" refers to a portion of the structure which is oriented towards a garment and is typically opposing the body facing surface and may be referred to as such. As used herein, the term "body surface" refers to a portion of an individual's body that the absorbent article is disposed with for collecting, absorbing, etc. fluid discharge from the individual.

The following discussion includes a description of the absorbent article, followed by a description of the method of use therefor in accordance with the present disclosure. Reference will now be made in detail to the exemplary embodiments of the disclosure, which are illustrated in the accompanying figures.

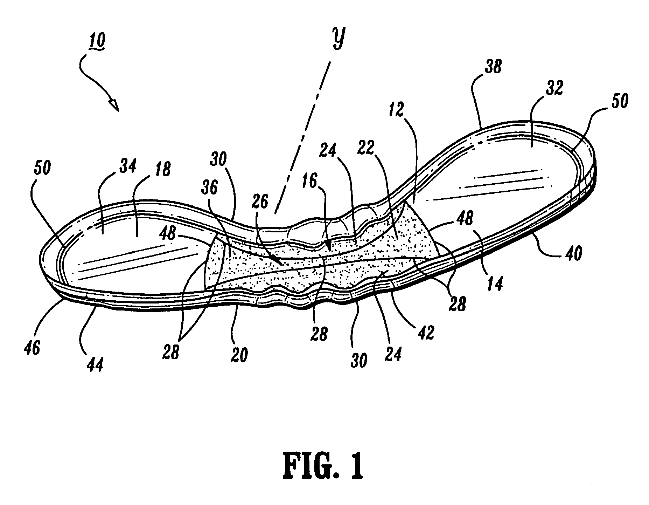

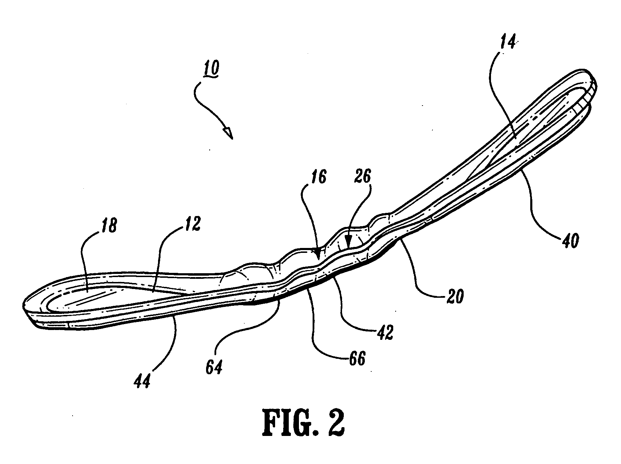

Turning now to the figures, wherein like components are designated by like reference numerals throughout the several views. Referring initially to FIGS. 1-3, there is illustrated an absorbent article 10, constructed in accordance with the principals of the present disclosure, including a fluid permeable top sheet 12. Absorbent article 10 further includes a first absorbent core 14 that is separate and in fluid communication with a second absorbent core 16. Second absorbent core 16 is separate and in fluid communication with a third absorbent core 18. First absorbent core 14, second absorbent core 16 and third absorbent core 18 are supported between top sheet 12 and a fluid impermeable back sheet 20.

Second absorbent core 16 defines a mid-section 22 and a pair of opposing lateral sections 24 which define a body facing surface 26 configured to engage top sheet 12. Absorbent article 10 is advantageously configured to prevent undesired product deformity, improving absorbency performance, and thereby avoiding premature leakage, overflow, etc. due to fluid discharge. Most advantageously, absorbent article 10 conforms to the contour of a body surface and allows for quick dispersal of fluid discharge through its fluid management features, as will be discussed below.

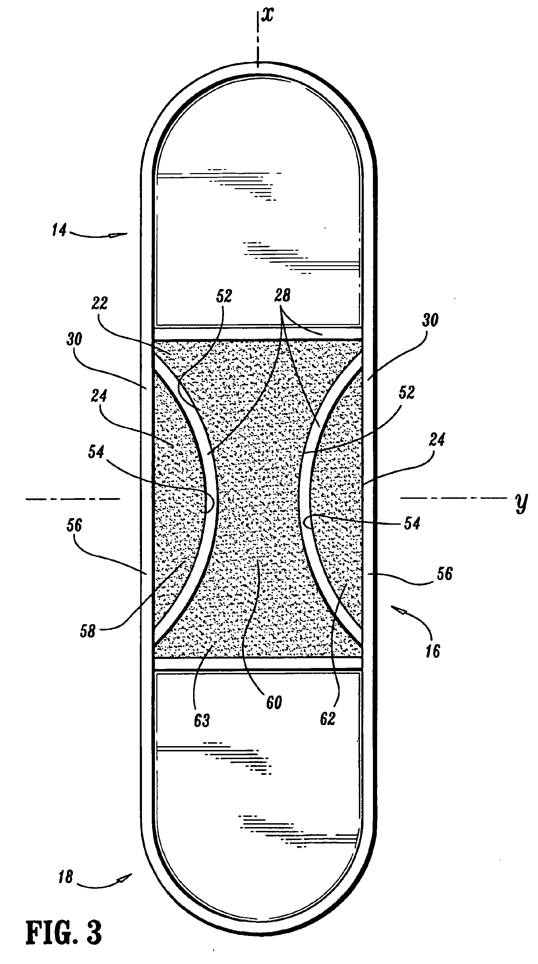

First absorbent core 14, second absorbent core 16 and third absorbent core 18 are disposed along a longitudinal length of absorbent article 10. As shown in FIG. 3, absorbent article 10 can be flattened to a planar configuration to define a longitudinal axis x. Absorbent core sections 22, 24 are disposed along an axis y substantially transverse to longitudinal axis x. Top sheet 12 and back sheet 20 define fluid permeable channels 28, as will be discussed, between absorbent cores 14, 16, 18 and absorbent core sections 22, 24. It is envisioned that channels 28 may be disposed between cores only, core sections only, or alternatively to separate predetermined areas of absorbent article 10 according to the requirements of a particular absorbent article application.

Channels 28 are configured to facilitate flexibility of absorbent article 10 relative to a body surface (not shown). Channels 28 advantageously distribute fluid flow outward to longitudinal sides 30 of absorbent article 10 to utilize more absorbent media of absorbent cores 14, 16, 18 to prevent leakage. The formation of channels 28 facilitates conformance of absorbent article 10 to the contour of the body surface and allows for quick dispersal of fluid discharge. This provides an absorbent article which folds in a desired configuration for comfort, improved absorbency protection and structural flexibility. It is contemplated that the orientation of channels 28 may alternatively be directed to various positions on absorbent article 10, such as, for example, an interior area, etc., according to the requirements of a particular absorbency application.

Absorbent article 10 is contemplated for fluid retention of discharged body fluids. More particularly, absorbent article 10 is envisioned to be a disposable absorbency device employing, among other things, absorbency and body conforming features to prevent leakage and overflow of fluids, as well as resistance to undesired deformity. The above advantages, among others, realized from the present disclosure are attained by the disclosed absorbent article 10, which is flexibly conforming to a body surface, as discussed herein below. These features of the present disclosure advantageously facilitate fluid retention of discharged fluids and prevent consequent overflow.

Top sheet 12 is disposed adjacent to a body facing surface 32 of absorbent core 14, body facing surface 26 of absorbent core 16 and a body facing surface 34 of absorbent core 18 and is configured to be worn against the body surface of the individual. In use, top sheet 12 is body fluid permeable, resilient, relatively non-absorbing and configured to direct fluid discharge to absorbent cores 14, 16, 18. Consequently, top sheet 12 is easily permeated by fluid discharge. Further, top sheet 12 retains minimal or no fluid in its structure to provide a relatively dry surface adjacent the body surface. It is also designed for comfort and conformability to an individual.

Top sheet 12 can be fabricated from a woven or non-woven, natural or synthetic material easily penetrated by fluid discharge. Top sheet 12 is a single sheet of material having a width sufficient to cover surfaces 26, 32 and 34. It is envisioned that top sheet 12 may include a multiple layer coverstock.

Top sheet 12 overlays and covers a greater surface area than absorbent cores 14, 16 and 18 to form top sheet outer edge 38. Outer edge 38 is sealed with back sheet 20 to fully enclose absorbent cores 14, 16 and 18. It is contemplated that top sheet 12 may be disposed over one or all of the absorbent cores used. It is further contemplated that top sheet 12 may be disposed over only a portion of a particular absorbent core. Top sheet 12 may be fabricated from fibers or filaments of thermoplastic polymers, such as, for example, polyethylene (PE), polypropylene (PP), bicomponent fibers (such as PE/PET or PE/PP), polyester (PET), etc. Top sheet 12 may also be made from other materials which allow the ready passage of fluid through to the absorbent core, as is known to one skilled in the art.

Back sheet 20 is disposed adjacent to a garment facing surface 40 of absorbent core 14, a garment facing surface 42 of absorbent core 16 and a garment facing surface 44 of absorbent core 18. Back sheet 20 generally faces away from the body surface and towards an undergarment worn by an individual.

Back sheet 20 permits passage of air and vapor from absorbent article 10 while preventing passage of fluid discharge therefrom. Back sheet 20 can be fabricated from a fluid impermeable material such as, for example, polymeric films such as polyethylene, polypropylene, cellophane, microporous films, SMS (spunbond-meltblown-spunbond), etc. or from a bi-component film such as ethel-vinyl-acetate polyethelyne coextruded film. A treated material may also be used such as impregnated fluid repellent paper or a non-woven fabric. Other materials, however, may be used as is known to one skilled in the art.

Back sheet 20 extends a sufficient surface area to include a back sheet outer edge 46 that attaches to top sheet outer edge 38 for enclosing absorbent cores 14, 16, 18. Outer edges 38, 40 may be joined by pressure sensitive adhesives, heat sensitive adhesives, ultrasonics or by other known joining applications which prevent fluid discharge flow beyond outer edges 38, 40 and, consequently, from absorbent article 10.

The sealing engagement of outer edges 38, 40 extends continuously around the periphery of absorbent article 10. It is contemplated however that the sealing engagement may be discontinuous, staggered etc. Outer edges 38, 40 have a soft, pliable configuration which is easily deformed by body movement and engagement with adjacent body areas, adding comfort quality to absorbent article 10. It is envisioned that outer edges 38, 40 may extend variable lengths from cores 14, 16, 18 or, alternatively, absorbent article 10 may not include edges 38, 40.

Absorbent cores 14, 18 are disposed on opposing sides of absorbent core 16 and connected thereto via channels 28. Absorbent cores 14, 18 are disposed longitudinally along absorbent article 10 relative to absorbent core 16 to facilitate placement adjacent a body surface. The longitudinal configuration of absorbent article 10 permits placement between an individuals' thigh area and can by drawn up to cover the crotch area.

Absorbent cores 14, 18 have linear edges 48 for alignment with corresponding surfaces of absorbent core 16. At their outer ends, absorbent cores 14, 18 have arcuate edges 50. Edges 48, 50 are also flexible for conforming to the body surface. It is envisioned that absorbent article 10, and absorbent cores 14, 16, 18 individually, may be manufactured in various configurations and dimensions, such as, for example, rectangular, oval, hourglass, etc. Absorbent cores 14, 16 and 18 are soft and configured for comfort to an individual.

Absorbent cores 14, 16 and 18 are fabricated from materials capable of absorbing and retaining fluid discharge, for example, a hydrophilic material such as cellulose fibers, wood pulp, re-generated cellulose, cotton fibers, cellulose acetate or a blend of pulp and other fibers. Bulk of absorbent article 10 can be reduced, due to the size of the absorbent cores, by adding superabsorbent polymer materials, having high liquid retention properties such as, for example, hydrocolloidal material, cross-linked acrylete polmers, etc., according to the requirements of a particular absorbency application. Super absorbent polymer particles can be permeated, desirably in granular form, through absorbent cores 14, 16, 18. It is contemplated that super absorbent polymer particles used are in the range of 3 to 8 grams, although other ranges are envisioned.

Surfaces 26 (as will be discussed), 32, 34 may include an acquisition layer(s) which aids in the transfer of fluid discharge to absorbent cores 14, 16, 18. This layer may include a tissue layer embossed or adhered to the absorbent cores. Other acquisition layers include Adhesive Bonded Polyester (ABPET), Through Air Bonded BiComponent (TABICO) fibers, 3-D aperatured films, 3-D aperatured Films and nonwoven (dual layer), high denier spunbond Polypropylene, chemical bonded nonwovens, etc. Absorbent cores 14, 16, 18 may also be chemically or physically modified. It is contemplated that the absorbent cores may include such materials in combination with other materials both natural and synthetic, such as airlaid materials, creeped cellulose wadding, melt blown polymers, tissue layers, tissue wraps, tissue laminates, foams, sponges, jelling material, etc.

Alternate designs are also envisioned whereby absorbent cores 14, 16, 18 may have varying caliper zones, hydrophilic gradients, super absorbent gradients, low-density acquisition zones, multiple layers or structures, etc., according to the particular requirements of an absorbent article application.

Second absorbent core 16 includes mid-section 22 and lateral sections 24. Mid-section 22 extends longitudinally along absorbent article 10. Mid-section 22 includes opposing concave sides 52 which are correspondingly aligned to convex sides 54 of lateral sections 24. Mid-section 22 is disposed adjacent lateral sections 24, in cooperation with top sheet 12 and back sheet 20, to form channels 28. Concave sides 52 and convex sides 54 define the boundaries of channels 28 and are substantially flexible. This configuration advantageously facilitates conformity to a body surface and prevents undesired deformity of absorbent article 10.

Channels 28 are configured to facilitate contour conformity to a body surface and desired deformity of absorbent article 10 relative to a body. Channels 28 distribute surplus fluid discharge flow to outer ends 38, 50 and therefore utilize more of absorbent core material as needed before failure can occur due to leakage. Channels 28 aid absorbent article 10 in achieving these advantages by moving with the body surface during wear. The configuration of channels 28 allows absorbent article 10 to quickly disperse fluids and perform effective fluid management.

Channels 28 include materials of the structural components which bound them, however, they may include other absorbent materials. It is envisioned that channels 28 may have various dimensions and configurations, such as, for example, circular, rectangular, polygonal cross-section, etc., according to the particular requirements of an absorbency application. It is further envisioned that channels 28 may be disposed adjacent various portions of absorbent article 10 or, alternatively, may be staggered, perforated, etc.

Absorbent article 10 includes elastic members 56 disposed with longitudinal sides 30. Elastic members 56 are configured to desirably deform the planar configuration of absorbent article 10. This desirable deformation resiliently pulls absorbent cores 14 and 18 toward each other relative to absorbent core 16. This resilient tendency of absorbent article 10 forms a cup or bucket shaped configuration, which is further facilitated by mid-section 22, lateral sections 24 and channels 28 formed therebetween. As elastic members 56 draw absorbent cores 14 and 18 toward the body surface, lateral sections 24 are also drawn towards the body surface relative to mid-section 22.

It is contemplated that varying lengths of elastic may be used. It is further contemplated that varying widths, individual strands or threads; round, square, or rectangular configurations, multiple strands grouped together, etc. may be used. The degree of elasticity, stiffness and flexibility of elastic members 56 may be altered according to the requirements of a particular absorbent article application. Elastic members 56 may also form elastic leg gathers to form a cuff-like shape about the thighs of an individual. The elastic members may be fabricated from any suitable material, such as, for example, synthetic or natural rubbers, such as heat sealable and heat shrinkable, latex, polyurethane, spandex, elastic foam, etc. It is contemplated that slot coating, spiral spray, meltblown or other adhesive applications may be used to secure elastic members 56 with absorbent article 10.

Elastic members 56 are disposed with longitudinal sides 30 to advantageously provide a reservoir, in connection with channels 28 due to the cup shape formed. These reservoirs provide absorbent barriers so that large fluid discharges can be managed by absorbent article 10. This configuration allows absorbent cores 14, 16 and 18 adequate time to perform fluid retention of the fluid discharge.

Elastic members 56, along with channels 28, define barriers between the separate absorbent cores 14, 16, 18 and separate core sections 22, 24 of absorbent core 16. This configuration provides spring like hinges within absorbent article 10 which results in a resistance to undesired deformity during use. Absorbent article 10 beneficially folds in a desired pad configuration providing comfort, improved absorbency protection and structural flexibility.

Separate absorbent core sections 22, 24 include three dimensional acquisition film layers 58, 60 and 62. Layer 60 is adhered to mid-section 22 and layers 58, 62 are adhered to lateral sections 24. Layer 60 is separated by channels 28 from layers 58, 62, similar to underlying sections 22, 24. As such, layers 58, 60, 62 cooperate with top sheet 12 to form channels 28. Layers 58, 60, 62 include apertures or cones 63 which facilitate fluid discharge flow to absorbent core 16. Thus, layers 58, 60, 62 manage, transport, accommodate and/or direct high volumes and high flow rates of fluid discharge to absorbent core 16. It is contemplated that layers 58, 60, 62 can be a through air bonded web, a bi-component non-woven web, cellulosic fibers, etc. Layers 58, 60, 62 may be adhesively secured in place by any suitable construction adhesive for absorbent core applications.

Back sheet 20 is a moisture barrier laminate positioned adjacent to garment facing surfaces 40, 42, 44 of absorbent cores 14, 16 and 18. Back sheet 20 includes a release strip 64 affixed thereto. Release strip 64 fixes absorbent article 10 to the outer crotch portion of an undergarment (not shown). Release strip 64 includes an adhesive element 66 which adheres back sheet 20 to the undergarment. It is envisioned that adhesive 66 may include a pressure sensitive adhesive material such as, for example, a water based adhesive such as, acrylic adhesives, etc. It is further envisioned that rapid setting thermoplastic adhesives, two-sided adhesive tape, adhesives based on natural or synthetic rubbers, etc. may be used. It is contemplated that adhesive 66 may include alternative shapes such as lines, squares, circles, etc.

In use, absorbent article 10 is properly prepared, sterilized and packaged for consumer application. Release strip 64 is removed to expose adhesive 66. Adhesive 66 is brought into engaging contact with an undergarment of an individual for attachment therewith. Absorbent article 10, with the undergarment, is disposed between the thighs of the individual. Absorbent cores 14 and 18 are oriented towards the front and the rear of the individual and absorbent core 16 is oriented directly below the crotch area. Absorbent cores 14 and 18 are drawn towards the body surface by elastic members 56. Channels 28 cooperate with elastic members 56 in a spring-like hinge arrangement to facilitate flexibility such that absorbent article 10 forms a cup-like configuration about the body surface. During use, channels 28 provide flexibility to absorbent article 10 to prevent undesired deformity thereof. Absorbent cores 14, 16, 18 absorb and retain fluid discharge. Other methods of use are also contemplated.

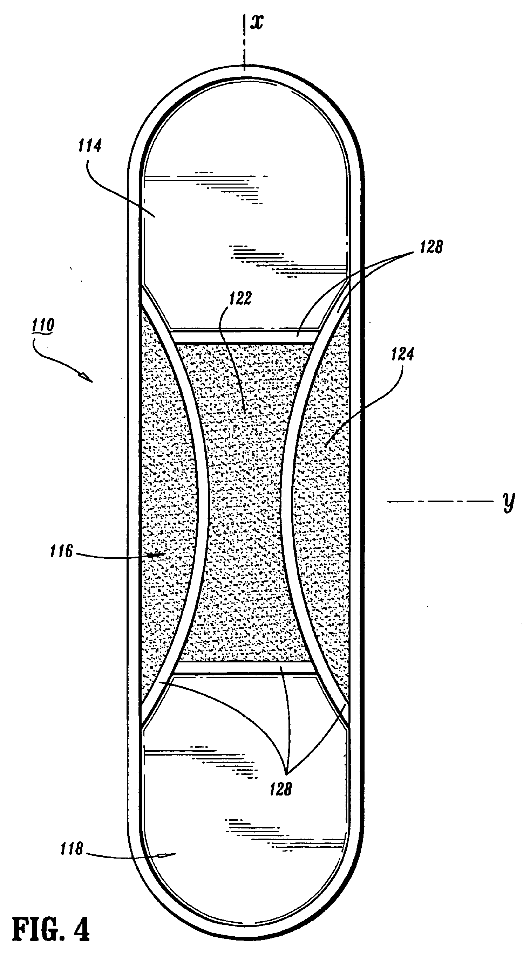

Referring to FIG. 4, an alternate embodiment of an absorbent article 110 is shown. Absorbent article 110, similar to absorbent article 10 discussed with regard to FIGS. 1-3, includes absorbent cores 114, 116, 118. Absorbent core 116 includes separate core sections 122, 124. Absorbent cores 114, 116 and 118 define fluid flow channels 128, similar to those described. Fluid flow channels 128, disposed along the arcuate surface of sections 124, extend longitudinally beyond the longitudinal length of mid-section 122. Thus, lateral sections 124 have a greater length than mid-section 122 and as such can accommodate a larger body surface area. This configuration advantageously provides greater flexibility to absorbent article 110, for example, in applications for individuals with larger thigh surface area, to prevent undesired deformity thereof.

## Claims

1. An absorbent article (110) comprising:
a fluid permeable top sheet (12);
a first absorbent core (114) being disposed adjacent and in fluid communication with a second absorbent core (116), the second absorbent core being disposed adjacent and in fluid communication with a third absorbent core (18,118), the first, second and third absorbent cores being arranged along a longitudinal axis of the absorbent article,
the first, second and third absorbent cores (114, 116, 118) being supported between the fluid permeable top sheet (12) and a fluid impermeable back sheet (20); and
the second absorbent core (116) defining three separate absorbent core sections (116, 122, 124) in fluid communication forming a central absorbent core section and two lateral absorbent core sections on each side of the central absorbent core section, the separate absorbent core sections defining a body facing surface that engages the fluid permeable top sheet (12),
**characterized in that** the first absorbent core (114) and the third absorbent core (118) are at first and second ends of the absorbent article (110) and wherein the top sheet (12) and the back sheet (20) define a first and second fluid permeable channels (128) between the absorbent cores, said first and second fluid permeable channels each extending between said central absorbent core section and a respective lateral absorbent core section and extending beyond a longitudinal length of the central absorbent core section.

2. An absorbent article as recited in claim 1, wherein the body facing surface includes an acquisition film.

3. An absorbent article as recited in claim 2, wherein the acquisition film has apertures or cones(63) formed therein to facilitate fluid flow.

4. An absorbent article as recited in claim 1, wherein the top sheet (12) and the back sheet (20) are joined to form longitudinal sides of the absorbent article, the absorbent cores (114, 116, 118) being disposed in alignment with the longitudinal sides.

5. An absorbent article as recited in claim 4, wherein the longitudinal sides include elastic members (56).

6. An absorbent article as recited in claim 1, wherein the permeable channels (128) are configured to facilitate flexibility of the absorbent article relative to a body.

7. An absorbent article as recited in claim 1, wherein the separate absorbent core sections 122, 124) include a mid-section having opposing concave sides and a pair of opposing lateral sections having convex sides positioned adjacent the opposing concave sides of the midsection.

8. An absorbent article as recited in claim 5, wherein the elastic members are configured to facilitate pivotal movement of the first absorbent core and the second absorbent core.

## Patentansprüche

1. Saugfähiger Artikel (110), der Folgendes umfasst:
eine fluiddurchlässige obere Lage (12);
einen ersten saugfähigen Kern (114), der sich angrenzend an einen zweiten saugfähigen Kern (116) befindet und damit in Fluidverbindung steht, wobei sich der zweite saugfähige Kern angrenzend an einen dritten saugfähigen Kern (18, 118) befindet und damit in Fluidverbindung steht, wobei der erste, der zweite und der dritte saugfähige Kern entlang einer Längsachse des saugfähigen Artikels angeordnet sind,
wobei der erste, der zweite und der dritte saugfähige Kern (114, 116, 118) zwischen der fluiddurchlässigen oberen Lage (12) und einer fluidundurchlässigen unteren Lage (20) abgestützt sind; und
der zweite saugfähige Kern (116) drei separate saugfähige Kernbereiche (116, 122, 124) definiert, die in Fluidverbindung einen zentralen saugfähigen Kernbereich und zwei seitliche saugfähige Kernbereiche auf jeder Seite des zentralen saugfähigen Kernbereichs bilden, wobei die separaten saugfähigen Kernbereiche eine dem Körper zugewandte Oberfläche definieren, die in die fluiddurchlässige obere Lage (12) eingreift,
**dadurch gekennzeichnet, dass** sich der erste saugfähige Kern (114) und der dritte saugfähige Kern (118) an einem ersten bzw. einem zweiten Ende des saugfähigen Artikels (110) befinden und die obere Lage (12) und die untere Lage (20) einen ersten bzw. einen zweiten fluiddurchlässigen Kanal (128) zwischen den saugfähigen Kernen definieren, wobei sich der erste und der zweite fluiddurchlässige Kanal jeweils zwischen dem zentralen saugfähigen Kernbereich und einem jeweiligen seitlichen saugfähigen Kernbereich sowie über eine in Längsrichtung verlaufende Länge des zentralen saugfähigen Kernbereichs erstrecken.

2. Saugfähiger Artikel nach Anspruch 1, bei dem die dem Körper zugewandte Oberfläche eine Aufnahmefolie beinhaltet.

3. Saugfähiger Artikel nach Anspruch 2, bei dem die Aufnahmefolie darin ausgebildete Öffnungen oder Kegel (63) hat, um ein Fließen von Fluid zu ermöglichen.

4. Saugfähiger Artikel nach Anspruch 1, bei dem die obere Lage (12) und die untere Lage (20) miteinander verbunden sind, um Längsseiten des saugfähigen Artikels zu bilden, wobei die saugfähigen Kerne (114, 116, 118) zu den Längsseiten hin ausgerichtet sind.

5. Saugfähiger Artikel nach Anspruch 4, bei dem die Längsseiten elastische Elemente (56) beinhalten.

6. Saugfähiger Artikel nach Anspruch 1, bei dem die durchlässigen Kanäle (128) so konfiguriert sind, dass eine Flexibilität des saugfähigen Artikels im Verhältnis zu einem Körper ermöglicht wird.

7. Saugfähiger Artikel nach Anspruch 1, bei dem die separaten saugfähigen Kernbereiche (122, 124) einen mittleren Bereich mit gegenüberliegenden konkaven Seiten und ein Paar gegenüberliegende seitliche Bereiche mit konvexen Seiten beinhalten, die angrenzend an die gegenüberliegenden konkaven Seiten des mittleren Bereichs positioniert sind.

8. Saugfähiger Artikel nach Anspruch 5, bei dem die elastischen Elemente so konfiguriert sind, dass eine Schwenkbewegung des ersten saugfähigen Kerns und des zweiten saugfähigen Kerns ermöglicht wird.

## Revendications

1. Article absorbant (110), comprenant:
une feuille supérieure perméable au fluide (12);
une première âme absorbante (114) qui est disposée à proximité et en communication de fluide avec une deuxième âme absorbante (116), la deuxième âme absorbante étant disposée à proximité de et en communication de fluide avec une troisième âme absorbante (18, 118), les première, deuxième et troisième âmes absorbantes étant agencées le long d'un axe longitudinal de l'article absorbant;
les première, deuxième et troisième âmes absorbantes (114, 116, 118) étant supportées entre la feuille supérieure perméable au fluide (12) et une feuille de dossier imperméable au fluide (20); et
la deuxième âme absorbante (116) définissant trois sections d'âme absorbante séparées (116, 122, 124) en communication fluidique qui forment une section d'âme absorbante centrale et deux sections d'âme absorbante latérales de part et d'autre de la section d'âme absorbante centrale, les sections d'âme absorbante séparées définissant une surface côté corps qui engage la feuille supérieure perméable au fluide (12),
**caractérisé en ce que** la première âme absorbante (114) et la troisième âme absorbante (118) se trouvent aux première et deuxième extrémités de l'article absorbant (110), et dans lequel la feuille supérieure (12) et la feuille de dossier (20) définissent des premier et deuxième canaux perméables au fluide (128) entre les âmes absorbantes, lesdits premier et deuxième canaux perméables au fluide s'étendant chacun entre ladite section d'âme absorbante centrale et une section d'âme absorbante latérale respective et s'étendant au-delà d'une longueur longitudinale de la section d'âme absorbante centrale.

2. Article absorbant selon la revendication 1, dans lequel la surface côté corps comprend un film de réception.

3. Article absorbant selon la revendication 2, dans lequel le film de réception comporte des ouvertures ou des cônes (63) qui sont formé(e)s dans celui-ci pour faciliter l'écoulement du fluide.

4. Article absorbant selon la revendication 1, dans lequel la feuille supérieure (12) et la feuille de dossier (20) sont jointes pour former des côtés longitudinaux de l'article absorbant, les âmes absorbantes (114, 116, 118) étant disposées en alignement avec les côtés longitudinaux.

5. Article absorbant selon la revendication 4, dans lequel les côtés longitudinaux comprennent des éléments élastiques (56).

6. Article absorbant selon la revendication 1, dans lequel les canaux perméables (128) sont configurés de manière à faciliter la flexibilité de l'article absorbant par rapport à un corps.

7. Article absorbant selon la revendication 1, dans lequel les sections d'âme absorbante séparées (122, 124) comprennent une section intermédiaire qui présente des côtés concaves opposés et une paire de sections latérales opposées qui présentent des côtés convexes qui sont positionnés à proximité des côtés concaves opposés de la section intermédiaire.

8. Article absorbant selon la revendication 5, dans lequel les éléments élastiques sont configurés de manière à faciliter un mouvement pivotant de la première âme absorbante et de la deuxième âme absorbante.
